(19) Europäisches Patentamt
European Patent Office
Office européen des brevets

(11) Veröffentlichungsnummer: **0 563 686 A1**

# (12) EUROPÄISCHE PATENTANMELDUNG

(21) Anmeldenummer: **93104387.1**

(22) Anmeldetag: **18.03.93**

(51) Int. Cl.5: **C07D 261/14**, A61K 31/42, C07D 413/04, A61K 31/44

(30) Priorität: **31.03.92 DE 4210502**

(43) Veröffentlichungstag der Anmeldung: **06.10.93 Patentblatt 93/40**

(84) Benannte Vertragsstaaten: **AT BE CH DE DK ES FR GB GR IT LI NL SE**

(71) Anmelder: **BAYER AG**

**D-51368 Leverkusen(DE)**

(72) Erfinder: **Jeschke, Peter, Dr.**
**Heymannstrasse 38**
**W-5090 Leverkusen 1(DE)**
Erfinder: **Lindner, Werner, Dr.**
**Märchenstrasse 39**
**W-5000 Köln 80(DE)**
Erfinder: **Harder, Achim, Dr. Dr.**
**Piccoloministrasse 398**
**W-5000 Köln 80(DE)**
Erfinder: **Mencke, Norbert, Dr.**
**Grunder Mühle 2**
**W-5090 Leverkusen 3(DE)**

## (54) Verwendung von 3-amino-substituierten Isoxazolderivaten zur Bekämpfung von Endoparasiten, neue 3-aminosubstituierte Isoxazolderivate und Verfahren zu ihrer Herstellung.

(57) Die vorliegende Erfindung betrifft die Verwendung von 3-aminosubstituierten Isoxazolderivaten der allgemeinen Formel (I)

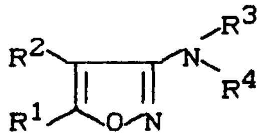

(I)

in welcher

$R^1$ für Aryl- oder Hetarylreste steht, die gegebenenfalls durch Halogen, Cyano, Nitro, Amino, Aminoacyl, Alkylamino, Alkyl, Aralkyl, Halogenalkyl, Alkoxy, Halogenalkoxy, Aryloxy, Arylthio, Halogenalkylthio, Alkylsulfinyl, Halogenalkylsulfinyl, Alkylsulfonyl, Halogenalkylsulfonyl substituiert sind,

$R^2$ für Wasserstoff, Halogen, Alkyl, Halogenalkyl, Cyano, Carbonylalkoxy, Aryl steht, die gegebenenfalls substituiert sind,

$R^3$ für substituiertes $C_1$-$C_6$-Alkyl, $C_2$-$C_4$-Alkenyl, sowie für gegebenenfalls substituiertes Cycloalkylalkyl, Cycloalkyl, Aralkyl oder Aryl steht, wobei als Substituenten genannt seien Halogen, Nitro, Alkyl, Halogenalkyl, Amino, Alkylamino, Aminoacyl, Alkoxy, Halogenalkoxy, Alkylthio, Halogenalkylthio, Alkylsulfinyl, Halogenalkylsulfinyl, Alkylsulfonyl, Halogenalkylsulfonyl, Aryl, Aryloxy, Arylthio,

$R^4$ für Wasserstoff, Alkyl steht oder $R^3$ und $R^4$ gemeinsam mit dem angrenzenden N-Atom für einen carbocyclischen 5-, 6- oder 7-gliedrigen Ring stehen, der gegebenenfalls auch durch O, S, N unterbrochen sein kann und gegebenenfalls durch $C_1$-$C_4$-Alkyl substituiert ist,

zur Bekämpfung von Endoparasiten in der Medizin und Tiermedizin, neue 3-aminosubstituierte Isoxazole und ihre Herstellung.

EP 0 563 686 A1

Die vorliegende Erfindung betrifft die Verwendung von 3-aminosubstituierten Isoxazolderivaten zur Bekämpfung von Endoparasiten, neue 3-aminosubstituierte Isoxazolderivate und Verfahren zu ihrer Herstellung.

3-Aminosubstituierte Isoxazolderivate sind bereits bekannt. Sie haben jedoch angeblich keine Wirkung gegen Endoparasiten (vgl. J.B. Carr et al. J. Med. Chem. 20 (1977), S. 934-939).

Die vorliegende Erfindung betrifft:

1. Die Verwendung von 3-aminosubstituierten Isoxazolderivaten der allgemeinen Formel (I)

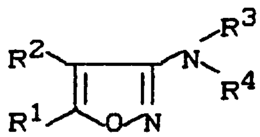

(I)

in welcher

$R^1$ für Aryl- oder Hetarylreste steht, die gegebenenfalls durch Halogen, Cyano, Nitro, Amino, Aminoacyl, Alkylamino, Alkyl, Aralkyl, Halogenalkyl, Alkoxy, Halogenalkoxy, Aryloxy, Arylthio, Halogenalkylthio, Alkylsulfinyl, Halogenalkylsulfinyl, Alkylsulfonyl, Halogenalkylsulfonyl substituiert sind,

$R^2$ für Wasserstoff, Halogen, Alkyl, Halogenalkyl, Cyano, Carbonylalkoxy, Aryl steht, die gegebenenfalls substituiert sind,

$R^3$ für substituiertes $C_1$-$C_6$-Alkyl, $C_2$-$C_4$-Alkenyl, sowie für gegebenenfalls substituiertes Cycloalkylalkyl, Cycloalkyl, Aralkyl oder Aryl steht, wobei als Substituenten genannt seien Halogen, Nitro, Alkyl, Halogenalkyl, Amino, Alkylamino, Aminoacyl, Alkoxy, Halogenalkoxy, Alkylthio, Halogenalkylthio, Alkylsulfinyl, Halogenalkylsulfinyl, Alkylsulfonyl, Halogenalkylsulfonyl, Aryl, Aryloxy, Arylthio,

$R^4$ für Wasserstoff, Alkyl steht oder $R^3$ und $R^4$ gemeinsam mit dem angrenzenden N-Atom für einen carbocyclischen 5-, 6- oder 7-gliedrigen Ring stehen, der gegebenenfalls auch durch O, S, N unterbrochen sein kann und gegebenenfalls durch $C_1$-$C_4$-Alkyl substituiert ist,

zur Bekämpfung von Endoparasiten in der Medizin und Tiermedizin.

Die Verbindungen der Formel (I) sind teilweise bekannt und lassen sich analog zu bekannten Verfahren herstellen.

2. Neue 3-aminosubstituierte Isoxazolderivate der allgemeinen Formel (I)

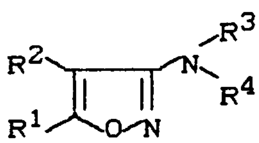

(I)

in welcher

$R^1$ für Arylreste die durch Alkoxy, Halogenalkoxy, Halogenalkylthio, Halogenalkylsulfinyl, Halogenalkylsulfonyl, sowie Hetarylreste, die gegebenenfalls durch Halogen, Alkyl, Halogenalkyl, Nitro, Alkoxy, Alkylthio substituiert sind,

$R^2$ für Wasserstoff, Halogen, Alkyl, Halogenalkyl, Cyano, Carbonylalkoxy, Aryl steht die gegebenenfalls substituiert sind,

$R^3$ für substituiertes $C_1$-$C_6$-Alkyl, $C_2$-$C_4$-Alkenyl, sowie für gegebenenfalls substituiertes Cycloalkylalkyl, Cycloalkyl, Aralkyl oder Aryl steht, wobei als Substituenten genannt seien Halogen, Nitro, Alkyl, Halogenalkyl, Amino, Alkylamino, Aminoacyl, Alkoxy, Halogenalkoxy, Alkylthio, Halogenalkylthio, Alkylsulfinyl, Halogenalkylsulfinyl, Alkylsulfonyl, Halogenalkylsulfonyl, Aryl, Aryloxy, Arylthio steht,

$R^4$ für Wasserstoff, Alkyl steht oder $R^3$ und $R^4$ gemeinsam mit dem angrenzenden N-Atom für einen carbocyclischen 5-, 6- oder 7-gliedrigen Ring stehen, der gegebenenfalls auch durch O, S, N unterbrochen sein kann und gegebenenfalls durch $C_1$-$C_4$-Alkyl substituiert ist,

ausgenommen 3-Ethylamino-5-(4-methoxyphenyl)-isoxazol.

3. Verfahren zur Herstellung der neuen 3-aminosubstituierten Isoxazolderivate der Formel (I)

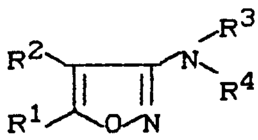

(I)

in welcher
$R^1$, $R^2$, $R^3$ und $R^4$ die unter Punkt 2 angegebene Bedeutung besitzen,
dadurch gekennzeichnet, daß man

a) für den Fall, daß $R^2$ ausgenommen für Halogen die oben angegebene Bedeutung hat, Verbindungen der Formel (II)

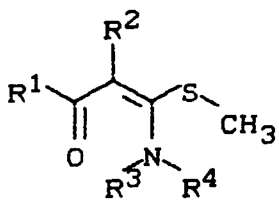

(II)

in welcher
$R^1$, $R^3$ und $R^4$ die oben angegebene Bedeutung haben,
mit Hydroxylamin umsetzt und die dabei zumeist in situ erhaltenen Verbindungen der Formel (III)

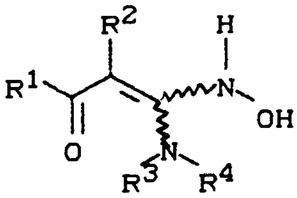

(III)

in welcher
$R^1$, $R^2$, $R^3$ und $R^4$ die oben angegebene Bedeutung haben,
in Gegenwart eines Verdünnungsmittels und gegebenenfalls in Gegenwart eines Reaktionshilfsmittels cyclisiert, oder

b) für den Fall, daß $R^2$ für Halogen steht, Verbindungen der Formel (Ia)

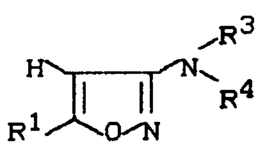

(Ia)

in welcher
$R^1$, $R^3$ und $R^4$ die unter Punkt 2 angegebene Bedeutung besitzen,
mit Halogenen der Formel (IV)

$Hal_2$ (IV)

in welcher
Hal für Halogen, vorzugsweise Fluor, Chlor, Brom oder Iod, insbesondere Brom, steht,
in Gegenwart eines Verdünnungsmittels umsetzt.

Die Verbindungen der Formel (I) eignen sich hervorragend zur Bekämpfung von Endoparasiten, besonders auf dem Gebiet der Veterinärmedizin.

Die erfindungsgemäßen 3-aminosubstituierten Isoxazolderivate sind durch die Formel (I) allgemein definiert.

Bevorzugt sind Verbindungen der Formel (I), in welcher

$R^1$ für substituiertes Phenyl, sowie für gegebenenfalls substituiertes Pyridinyl, Furanyl, Thiophenyl steht, wobei als Substituenten genannt seien eine oder mehrere gleiche oder verschiedene Reste der Gruppe $C_1$-$C_4$-Alkyl, insbesondere Methyl, Ethyl oder Isopropyl, $C_1$-$C_4$-Alkoxy, insbesondere Methoxy, Ethoxy, Methylendioxy oder Ethylendioxy, die gegebenenfalls durch Fluor oder Chlor substituiert sind, $C_1$-$C_4$-Halogenalkoxy, insbesondere Trifluormethoxy, Fluorchlorethoxy, $C_1$-$C_4$-Alkylthio, insbesondere Methylthio, $C_1$-$C_4$-Halogenalkylthio, insbesondere Trifluormethylthio, Fluorchlormethylthio, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Halogen, insbesondere Fluor, Chlor oder Brom, Nitro, Cyano, Amino, $C_1$-$C_4$-Alkylamino, $C_1$-$C_4$-Dialkylamino, insbesondere Dimethylamino, $C_3$-$C_6$-Cycloalkylamino, insbesondere Piperidino, $C_1$-$C_4$-Cycloalkoxyamino, insbesondere Morpholino, Acylamino, insbesondere Acetylamino, Phenyl, Phenylthio, Phenoxy, die gegebenenfalls durch einen oder mehrere gleiche oder verschiedene der oben angegebenen Reste substituiert sind,

$R^2$ für Wasserstoff, Halogen, vorzugsweise Chlor oder Brom, insbesondere Brom, $C_1$-$C_4$-Alkyl, insbesondere Methyl, Ethyl oder Isopropyl, $C_1$-$C_4$-Halogenalkyl, insbesondere Trifluormethyl, Cyano, Carbonyl-$C_1$-$C_4$-alkoxy, insbesondere Carbonylmethoxy oder Carbonylethoxy, Phenyl das gegebenenfalls durch einen der oben angegebenen Reste substituiert ist,

$R^3$ für substituiertes $C_1$-$C_6$-Alkyl, $C_2$-$C_4$-Alkenyl, $C_3$-$C_6$-Cycloalkyl-$C_1$-$C_2$-alkyl, $C_3$-$C_6$-Cycloalkyl, Phenyl-$C_1$-$C_2$-alkyl und Phenyl steht, die gegebenenfalls durch einen oder mehrere gleiche oder verschiedene der oben angegebenen Reste substituiert sind,

$R^4$ für Wasserstoff, $C_1$-$C_6$-Alkyl steht oder $R^3$ und $R^4$ gemeinsam mit dem angrenzenden N-Atom für einen carbocyclischen 5-, 6- oder 7-gliedrigen Ring stehen, der gegebenenfalls auch durch O, S, N unterbrochen sein kann und gegebenenfalls durch $C_1$-$C_4$-Alkyl substituiert ist.

Besonders bevorzugt sind Verbindungen der Formel (I)

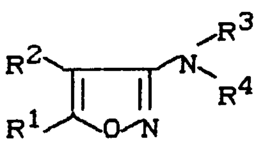

(I)

in welcher

$R^1$ die weiter oben angegebene Bedeutung besitzt,

$R^2$ für Wasserstoff, Halogen wie Brom, $C_1$-$C_4$-Alkyl wie Methyl, Ethyl oder Isopropyl steht,

$R^3$ für substituiertes $C_1$-$C_6$-Alkyl, insbesondere Methyl, Ethyl, Propyl, Isopropyl, Butyl, Isobutyl, sec.-Butyl, tert.-Butyl, $C_2$-$C_4$-Alkenyl, insbesondere Allyl, Cyclopropyl-methyl, Cyclopropyl, Cyclopentyl, Cyclohexyl, Benzyl, 1-Phenyl-ethyl und Phenyl steht, die gegebenenfalls durch einen oder mehrere gleiche oder verschiedene der oben angegebenen Reste substituiert sind,

$R^4$ für Wasserstoff steht.

Ganz besonders bevorzugt sind Verbindungen der Formel (I), in welcher

$R^1$ für Phenyl, Pyridinyl, Furanyl, Thiophenyl steht, die gegebenenfalls substituiert sind durch Wasserstoff, Halogen, insbesondere Fluor, Chlor oder Brom, $C_1$-$C_4$-Alkyl, insbesondere Methyl, Ethyl oder Isopropyl, $C_1$-$C_4$-Halogenalkyl, insbesondere Trifluormethoxy, $C_1$-$C_4$-Alkylthio, insbesondere Methylthio, $C_1$-$C_4$-Halogenalkylthio, insbesondere Trifluormethylthio steht,

$R^2$ für Wasserstoff, Halogen wie Brom, $C_1$-$C_4$-Alkyl wie Methyl oder Ethyl steht,

$R^3$ für substituiertes $C_1$-$C_6$-Alkyl, insbesondere Methyl, Ethyl, Propyl, Isopropyl, Butyl, Isobutyl, sec.-Butyl, tert.-Butyl, $C_2$-$C_4$-Alkenyl, insbesondere Allyl, Cyclopropyl-methyl, Cyclopropyl, Cyclopentyl, Cyclohexyl, Benzyl, 1-Phenyl-ethyl steht und

$R^4$ für Wasserstoff steht.

Im einzelnen seien folgende Verbindungen der Formel (I) genannt, in welcher die Reste $R^1$, $R^2$, $R^3$ und $R^4$ die folgende Bedeutung haben:

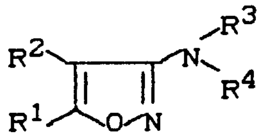

(I)

5-(Pyrid-4-yl)-3-cyclopropyl-methylamino-isoxazol,
5-(Pyrid-4-yl)-3-phenyl-ethylamino-isoxazol,
5-(Pyrid-3-yl)-3-cyclopropyl-methylamino-isoxazol,
5-(Pyrid-3-yl)-3-phenyl-ethylamino-isoxazol,
5-(Pyrid-3-yl)-3-sec.-butylamino-isoxazol,
5-(Pyrid-3-yl)-3-tert.-butylamino-isoxazol,
5-(Pyrid-3-yl)-3-isopropylamino-isoxazol,
5-(2,4,6-Trimethyl-pyrid-3-yl)-3-cyclopropyl-methylamino-isoxazol,
5-(2,4,6-Trimethyl-pyrid-3-yl)-3-phenyl-ethylamino-isoxazol,
5-(2,3-Dichlor-pyrid-3-yl)-3-cyclopropyl-methylamino-isoxazol,
5-(Thien-3-yl)-3-cyclopropyl-methylamino-isoxazol,
5-(Thien-3-yl)-3-phenyl-ethylamino-isoxazol,
5-(Thien-3-yl)-3-cyclopropylamino-isoxazol,
5-(5-Chlor-thien-3-yl)-3-cyclopropyl-methylamino-isoxazol,
5-(4-Isopropyl-thien-2-yl)-3-isopropylamino-isoxazol,
5-(2,5-Dimethyl-thien-3-yl)-3-cyclopropylamino-isoxazol,
5-(Fur-2-yl)-3-cyclopropyl-methylamino-isoxazol,
5-(4-Trifluormethylthio-phenyl)-3-cyclopropyl-methylamino-isoxazol,
5-(4-Trifluormethylthio-phenyl)-3-sec.-butylamino-isoxazol.

Von den neuen Verbindungen der Formel (I) sind diejenigen bevorzugt und besonders bevorzugt in denen die Substituenten die oben angegebenen bevorzugten Definitionen besitzen.

Die Verbindungen der Formel (I) sind teilweise bekannt, sie lassen sich nach den oben unter Punkt 3 angegebenen Verfahren a) bis b) herstellen (vgl. z.B. EP 0 181 018; S. Sugai et al. Chem. Pharm. Bull. 27 (1979), S. 2787 bis 2794; H. Junjappa et al. Synthesis (1984), S. 247 bis 249; R.K. Dieter et al. J. Org. Chem. 54 (1989), S. 1088 bis 1092; W.-D. Rudorf et al. J. prakt. Chem. 320 (1978), S. 585 bis 599; A. Dornow et al. Chem. Ber. 100 (1967), S. 2577 bis 2584; Houben-Weyl, Methoden der organischen Chemie, Bd. E 11, 4.Aufl. 1985, G. Thieme Verlag, Stuttgart-New York, S. 339).

Setzt man bei Verfahren 3a zur Herstellung der neuen 3-aminosubstituierten Isoxazolderivate als Verbindungen der Formel (II) (E)-1-(2,5-Dichlor-thien-2-yl)-3-ethylamino-3-methylthio-1-oxo-prop-2-en ein, so läßt sich das Verfahren durch folgendes Reaktionsschema wiedergeben:

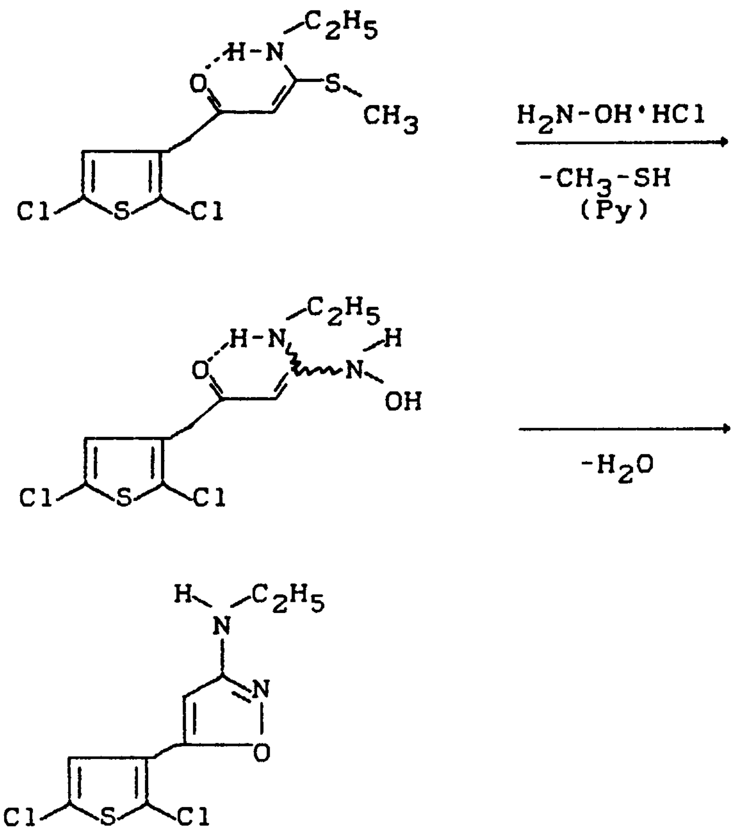


Die zur Durchführung des erfindungsgemäßen Verfahrens 3a als Ausgangsstoffe benötigten (E)-1-Hetaryl-(Aryl)-3-alkyl(aryl)amino-3-methylthio-1-oxo-prop-2-ene sind durch die Formel (II) allgemein definiert. In dieser Formel stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten Verbindungen der Formel (II) sind teilweise bekannt (vgl. z.B. F.C.V. Larsson et al, Tetrahedron 30 (1974), S. 1283 bis 1288; H. Junjappa et al. Synthesis (1980), S. 748 bis 751; W.-D. Rudorf et al. Tetrahedron 35 (1979), S. 551 bis 556; W. Schroth et al. Synthesis (1982), S. 203; Houben-Weyl, Methoden der organischen Chemie, Bd. E 11 4,Aufl` 1985, G.Thieme Verlag, Stuttgart-New York, S. 438) bzw. können nach den dort beschriebenen Verfahren erhalten werden.

Die Verbindungen der Formel (II) und (III) können als geometrische Isomere oder Isomerengemische unterschiedlicher Zusammensetzung vorliegen.

Im einzelnen seien folgende Verbindungen der Formel (II) genannt, in welcher die Reste $R^1$, $R^2$, $R^3$ und $R^4$ die folgende Bedeutung haben:

(E)-1-(Pyrid-4-yl)-3-ethylamino-3-methylthio-1-oxo-prop-2-en,
(E)-1-(Pyrid-3-yl)-3-ethylamino-3-methylthio-1-oxo-prop-2-en,
(E)-1-(Pyrid-2-yl)-3-ethylamino-3-methylthio-1-oxo-prop-2-en,
(E)-1-(2,4,6-Trimethyl-pyrid-3-yl)-3-ethylamino-3-methylthio-1-oxo-prop-2-en,
(E)-1-(2,3-Dichlor-pyrid-5-yl)-3-ethylamino-3-methylthio-1-oxo-prop-2-en,
(E)-1-(Thien-2-yl)-3-ethylamino-3-methylthio-1-oxo-prop-2-en,
(E)-1-(Thien-3-yl)-3-ethylamino-3-methylthio-1-oxo-prop-2-en,
(E)-1-(5-Chlor-thien-3-yl)-3-ethylamino-3-methylthio-1-oxo-prop-2-en,
(E)-1-(5-Brom-thien-2-yl)-3-ethylamino-3-methylthio-1-oxo-prop-2-en,
(E)-1-(4-Brom-thien-2-yl)-3-ethylamino-3-methylthio-1-oxo-prop-2-en,
(E)-1-(2,5-Dimethyl-thien-3-yl)-3-ethylamino-3-methylthio-1-oxo-prop-2-en,
(E)-1-(5-Methyl-thien-2-yl)-3-ethylamino-3-methylthio-1-oxo-prop-2-en,
(E)-1-(4-Isopropyl-thien-2-yl)-3-ethylamino-3-methylthio-1-oxo-prop-2-en,
(E)-1-(Fur-2-yl)-3-ethylamino-3-methylthio-1-oxo-prop-2-en,
(E)-1-(5-Methyl-fur-2-yl)-3-ethylamino-3-methylthio-1-oxo-prop-2-en.

Die Reaktion der Verbindungen der Formel (II) wird vorzugsweise unter Verwendung von Verdünnungsmitteln und in Gegenwart eines basischen Reaktionshilfsmittels durchgeführt.

Als Verdünnungsmittel zur Durchführung des erfindungsgemäßen Verfahrens 3a kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylether, Di-n-butylether, Di-isobutylether, Di-isoamylether, Di-isopropylether, Anisol, Phenetol, Cyclohexylmethylether, Diethylether, Ethylenglykoldimethylether, Tetrahydrofuran, Dioxan, Dichlordiethylether, Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische, cycloaliphatische oder aromatische Kohlenwasserstoffe wie Heptan, Hexan, Nonan, Cymol, Benzinfraktionen innerhalb eines Siedepunktintervalles von 70 °C bis 190 °C, Cyclohexan, Methylcyclohexan, Petrolether, Ligroin, Octan, Benzol, Toluol, Xylol; Ester wie Ethylacetat, Isobutylacetat; Amide z.B. Formamid, N-Methylformamid, N,N-Dimethylformamid, N-Methyl-pyrrolidon; Ketone wie Aceton, Methylethylketon. Auch Gemische der genannten Lösungs- und Verdünnungsmittel kommen in Betracht.

Bevorzugt sind Alkohole wie Methanol und Ethanol.

Als basische Reaktionshilfsmittel können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Oxide und Carbonate des Lithiums, Natriums, Kaliums, Magnesiums, Calciums und Bariums, ferner weitere basische Verbindungen wie Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methylpiperidin, N-Methyl-imidazol, N-Methyl-pyrrol, N-Methyl-morpholin, N-Methyl-hexamethylenimin, Pyridin, Chinolin, α-Picolin, β-Picolin, Isochinolin, Pyrimidin, Acridin, N,N,N',N'-Tetra-methylendiamin, N,N,N',N'-Tetra-ethylendiamin, Chinoxalin, N-Propyl-diisopropylamin, N,N'-Dimethyl-cyclohexylamin, 2,6-Lutidin, 2,4-Lutidin, Triethylen-diamin, Diazabicyclooctan (DABCO), Diazabicyclononen (DBN) oder Diazabicycloundecen (DBU).

Vorzugsweise verwendet man Heteroaromaten, wie beispielsweise Pyridin oder Hydroxide des Kaliums oder Natriums.

Das Verfahren 3a wird durchgeführt, indem man Verbindungen der Formel (II) mit einem Überschuß an Hydroxylamin-hydrochlorid zusammengibt und in Gegenwart eines basischen Reaktionshilfsmittels erhitzt. Hierbei ist das basische Reaktionshilfsmittel zugleich Verdünnungsmittel. Die Reaktionsdauer beträgt ca. 1 bis 8 Stunden. Die Reaktion wird bei Temperaturen zwischen +20 °C und +200 °C, bevorzugt zwischen +70 °C und +155 °C durchgeführt. Vorzugsweise arbeitet man unter einer Inertgasatmosphäre und bei dem Druck, der sich beim Erhitzen auf die erforderliche Reaktionstemperatur unter den Reaktionsbedingungen einstellt.

Zur Durchführung des erfindungsgemäßen Verfahrens setzt man pro Mol an (E)-1-Hetaryl(Aryl)-3-alkyl(aryl)amino-3-methylthio-1-oxo-prop-2-en der Formel (II) im allgemeinen 1,0 bis 3,0 Mol, vorzugsweise 1,5 bis 2,5 Mol, an Hydroxylamin-hydrochlorid ein.

Nach vollendeter Umsetzung wird das Reaktionsgemisch abgekühlt, der gesamte Reaktionsansatz in Wasser eingetragen, mit einem organischen Lösungsmittel extrahiert und in an sich bekannter Weise aufgearbeitet. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 3a zur Herstellung der neuen 3-aminosubstituierten Isoxazolderivate als Verbindungen der Formel (Ia) 5-(Thien-2-yl)-ethylamino-isoxazol ein, so läßt sich das Verfahren durch folgendes Reaktionsschema wiedergeben:

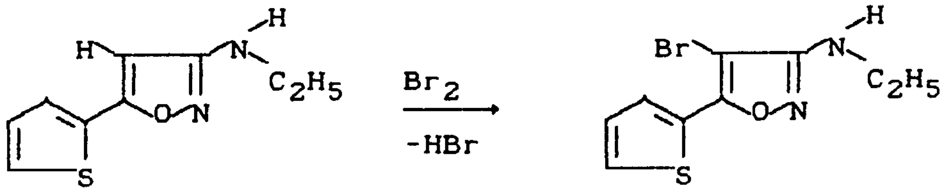


Die zur Durchführung des erfindungsgemäßen Verfahrens 3a als Ausgangsstoffe benötigten 5-Hetaryl(Aryl)-3-alkyl(aryl)amino-isoxazole sind durch die Formel (Ia) allgemein definiert. In dieser Formel stehen $R^1$, $R^3$

und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die außerdem für die Durchführung des erfindungsgemäßen Verfahrens 3b als Ausgangsstoffe zu verwendenden Halogene der Formel (IV) sind allgemein bekannte Verbindungen der organischen Chemie.

Die Halogenierung 5-substituierter Isoxazole in 4-Position ist bekannt (vgl. A.N. Nesmeyanov et al. Khim. Geterotsikl. Soedin. 5 (1967) S. 800 bis 805; ref.: C.A. 104 (1968) 557; Zh. Obshch. Khim. 28 (1958) S. 359 bis 262; J.B. Carr et al. J. Med, Chem. 20 (1977) S. 934 bis 939; EP 0 181 018) und kann nach den dort beschriebenen Verfahren durchgeführt werden. Alternativ können anstelle der Halogene auch Halogenierungsreagenzien wie beispielsweise Sulfurylhalogenide (US-Pat. 3 879 532) eingesetzt werden.

Die Reaktion der Verbindungen der Formel (Ia) und (IV) wird vorzugsweise unter Verwendung von Verdünnungsmitteln und gegebenenfalls in Gegenwart eines basischen Reaktionshilfsmittels durchgeführt.

Als Verdünnungsmittel zur Durchführung des Verfahrens 3b finden die bei Verfahren 3a genannten Halogenkohlenwasserstoffe, insbesondere Chlorkohlenwasserstoffe Verwendung.

Das Verfahren 3b wird durchgeführt, indem Verbindungen der Formel (Ia) und ein geringer Überschuß der Verbindungen der Formel (IV) in einem der angegebenen Verdünnungsmittel zusammengegeben und gerührt werden. Die Reaktionsdauer beträgt 10 Minuten bis 24 Stunden. Die Umsetzung erfolgt bei Temperaturen zwischen -5 °C und +100 °C, bevorzugt zwischen -5 °C und +50 °C, besonders bevorzugt bei 0 °C bis Raumtemperatur. Es wird unter Normaldruck gearbeitet.

Zur Durchführung des erfindungsgemäßen Verfahrens 3b setzt man pro Mol an 5-(Thien-2-yl)-3-ethylamino-isoxazol der Formel (Ia) im allgemeinen 1,0 bis 3,0 Mol, vorzugsweise 1,0 bis 1,5 Mol an Halogenverbindung der Formel (IV) ein.

Nach vollendeter Umsetzung wird gegebenenfalls im Vakuum eingeengt, der ausgefallene Feststoff abfiltriert, gewaschen und getrocknet. Die anfallenden Produkte lassen sich in üblicher Weise durch Umkristallisieren, Vakuumdestillation oder Säulenchromatographie reinigen (vgl. auch die Herstellungsbeispiele).

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsmindetungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stileasia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystome spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragoniums spp., Collyriclum spp., Nanophyetus spp., Opisthorchis spp., Clonorchis spp., Metorchis spp., Heterophyes spp., Metagonimus spp..

Aus der Ordnung der Enoplida z.B.: Trichuris spp., Capillaria spp., Trichomosoides spp., Trichinella spp.

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp.,

Trichostrongylus spp., Haemonchus spp., Ostertagia spp., Mashallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Monoliformis spp., Macracanthorhynchus spp., Prosthenorchis spp..

Zu den Nutz- und Zuchttieren gehören Säugetiere wie z.B. Kinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldharnster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zur Injektion; Halbfeste Zubereitungen;

Formulierungen, bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele wrden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systematisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glykole, Polyethylenglykole, Polypropylenglykole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmonobutylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Siliconöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Siliconöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitanmonooleat, Sorbitanmonostearat, Glycerinmonostearat, Polyoxyethylstearat, Alkylphenolpolyglykolether;
ampholytische Tenside wie Di-Na-N-lauryl-$\beta$-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit, gegebenenfalls unter Zusatz weiterer Hilfsstoffe wie Netzmittel, Farbstoffe, resorptionsfördernde Stoffe, Konservierungsstoffe, Antioxidantien, Lichtschutzmittel, suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen, gegebenenfalls unter Zusatz von Hilfsstoffen, vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 bis 10 Gewichtsprozent.

Zubereitungen, die vor Anwendung verdünnt werden, enthalten den Wirkstoff in Konzentrationen von 0,5 bis 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

## Beispiel A

In vivo Nematodentest

Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 4 | 10 |
| 6 | 10 |
| 10 | 10 |
| 22 | 10 |
| 41 | 10 |
| 57 | 10 |
| 63 | 10 |

Beispiel B

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Wärmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 4 | 10 |
| 6 | 10 |
| 10 | 10 |
| 13 | 10 |
| 22 | 10 |
| 43 | 10 |
| 57 | 10 |

Herstellungsbeispiele

Beispiel 1

5-(2,5-Dichlor-thien-3-yl)-3-ethylamino-isoxazol

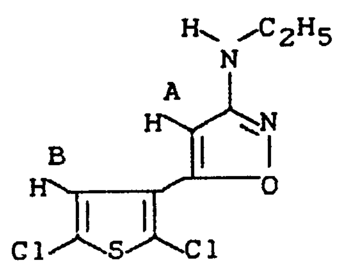

25,0 g (84,4 mmol) (E)-1-(2,5-Dichlor-thien-3-yl)-3-ethylamino-3-methylthio-1-oxo-prop-2-en werden in 13,5 ml absolutem Pyridin vorgelegt und mit 11,7 g (168,3 mmol) Hydroxylamin-hydrochlorid versetzt (Inertgasatmosphäre). Die Mischung wird bis zum vollständigen Umsatz (ca. 2 Stunden) bei Rückflußtemperatur gerührt. Danach wird der gesamte Reaktionsansatz in Wasser eingetragen, mit Essigsäureethylester extrahiert und mit 2N Salzsäure angesäuert. Die organische Phase wird abgetrennt, mit Wasser gewaschen, über Natriumsulfat getrocknet und anschließend das Lösungsmittel abdestilliert. Das zurückbleibende Rohprodukt kann über eine Kieselgelsäule (Kieselgel 60-Merck, Korngröße: 0,040 bis 0,063 mm) mit dem Fließmittel Toluol : Essigsäureethylester (20:1) chromatographiert werden.

Man erhält 7,2 g (32,4 % der Theorie) 5-(2,5-Dichlorthien-3-yl)-3-ethylamino-isoxazol.

Fp.: 65-66 ° C.

$^{1}$H-NMR (DMSO-$d_6$, $\delta$): 1,15 (t, 3H, $-CH_3$; H: 7,2 Hz); 3,11 (m, 2H, $-CH_2-$; J: 7,2 Hz); 6,26 (t br., 1H, NH; J: 5,5 Hz); 6,37 (s, 1H, $=CH^A-$); 7,51 (s, 1H, $=CH^B-$) ppm

Analog können die in der nachstehenden Tabelle 1 aufgeführten Verbindungen der Formel (I) hergestellt werden.

Tabelle 1

Beispiele für die Verbindungen der Formel (I)

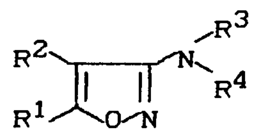

(I)

| Bsp. Nr. | $R^1$ | $R^2$ | $R^3$ | $R^4$ | Physikalische Daten |
|---|---|---|---|---|---|
| 2 | Phenyl | H | H | $C_2H_5$ | Fp.: 104-105° C |
| 3 | 4-$OCH_3$-Phenyl | H | H | $CH_3$ | Fp.: 87-88° C |
| 4 | 4-$OCH_3$-Phenyl | H | H | $C_2H_5$ | Fp.: 74-76° C |
| 5 | 4-$OCH_3$-Phenyl | H | H | i-$C_3H_7$ | Fp.: 98-99° C |
| 6 | 4-$OCH_3$-Phenyl | H | H | c-$C_3H_5$ | Fp.: 89-90° C |
| 7 | 4-$OCH_3$-Phenyl | H | H | i-$C_4H_9$ | Fp.: 76-77° C |
| 8 | 4-$OCH_3$-Phenyl | H | H | s-$C_4H_9$ | Fp.: 78-79° C |
| 9 | 4-$OCH_3$-Phenyl | H | H | t-$C_4H_9$ | Fp.: 109-110° C |
| 10 | 4-$OCH_3$-Phenyl | H | H | $CH_2$-c-$C_3H_5$ | Fp.: 92-93° C |

Tabelle 1 - Fortsetzung

| Bsp. Nr. | $R^1$ | $R^2$ | $R^3$ | $R^4$ | Physikalische Daten |
|---|---|---|---|---|---|
| 11 | 4-$OCH_3$-Phenyl | H | H | c-$C_6H_{11}$ | Fp.: 122-124° C |
| 12 | 4-$OCH_3$-Phenyl | H | H | $CH_2$-Phenyl | Fp.: 118-119° C |
| 13 | 4-$OCH_3$-Phenyl | H | H | $(CH_2)_2$-Phenyl | Fp.: 106-107° C |
| 14 | 4-$OCH_3$-Phenyl | $CH_3$ | H | $C_2H_5$ | Fp.: 65-66° C |
| 15 | 4-$OCH_3$-Phenyl | $C_2H_5$ | H | $C_2H_5$ | Fp.: 64-65° C |
| 16 | 4-$OCH_3$-Phenyl | 4-$OCH_3$-Phenyl | H | $C_2H_5$ | Fp.: 106-107° C |
| 17 | 3-$OCH_3$-Phenyl | H | H | $C_2H_5$ | Fp.: 57-58° C |
| 18 | 2-$OCH_3$-Phenyl | H | H | $C_2H_5$ | Fp.: 117-118° C |
| 19 | 3,4-$(OCH_3)_2$-Phenyl | H | H | $C_2H_5$ | Fp.: 92-93° C |
| 20 | 3-$CH_3$, 4-$OCH_3$-Phenyl | H | H | $C_2H_5$ | Fp.: 45-47° C |
| 21 | 4-$OCF_3$-Phenyl | H | H | $C_2H_5$ | Fp.: 101-103° C |
| 22 | 4-$SCF_3$-Phenyl | H | H | $C_2H_5$ | Fp.: 139-141° C |
| 23 | 4-$SCF_3$-Phenyl | H | H | c-$C_6H_{11}$ | Fp.: 96-100° C |

Tabelle 1 - Fortsetzung

| Bsp. Nr. | $R^1$ | $R^2$ | $R^3$ | $R^4$ | Physikalische Daten |
|---|---|---|---|---|---|
| 24 | 4-$SCH_3$-Phenyl | H | H | $C_2H_5$ | Fp.: 95-96° C |
| 25 | 3-O-Benzyl-Phenyl | H | H | $C_2H_5$ | Fp.: 181-183° C |
| 26 | 4-$CH_3$-Phenyl | H | H | $C_2H_5$ | Fp.: 115-116° C |
| 27 | 3,4-$(CH_3)_2$-Phenyl | H | H | $C_2H_5$ | Fp.: 71-73° C |
| 28 | 4-Phenyl-Phenyl | H | H | $C_2H_5$ | Fp.: 133-135° C |
| 29 | 4-i-$C_3H_7$-Phenyl | H | H | $C_2H_5$ | Fp.: 71-72° C |
| 30 | 4-$N(CH_3)_2$-Phenyl | H | H | $C_2H_5$ | Fp.: 149-151° C |
| 31 | 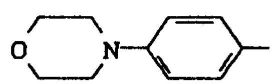 | H | H | $C_2H_5$ | Fp.: 175-176° C |
| 32 | t-$C_4H_9$ 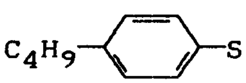 S- | H | H | $C_2H_5$ | Fp.: 85-86° C |
| 33 | 3-$CF_3$-Phenyl | H | H | $C_2H_5$ | Fp.: 69-71° C |

Tabelle 1 - Fortsetzung

| Bsp. Nr. | $R^1$ | $R^2$ | $R^3$ | $R^4$ | Physikalische Daten |
|---|---|---|---|---|---|
| 34 | 3,5-$(CF_3)_2$-Phenyl | H | H | c-$C_6H_{11}$ | Fp.: 109-111° C |
| 35 | 4-Br-Phenyl | H | H | $CH_3$ | Fp.: 157-160° C |
| 36 | 4-Br-Phenyl | H | H | $C_2H_5$ | Fp.: 153-154° C |
| 37 | 4-Br-Phenyl | H | H | c-$C_3H_5$ | Fp.: 140-142° C |
| 38 | 4-Br-Phenyl | H | H | $CH_2$-c-$C_3H_5$ | Fp.: 120-122° C |
| 39 | 4-Br-Phenyl | H | H | $CH_2$-CH=$CH_2$ | Fp.: 114-116° C |
| 40 | 4-Br-Phenyl | H | H | i-$C_4H_9$ | Fp.: 108-110° C |
| 41 | 4-Br-Phenyl | H | H | i-$C_4H_9$ | Fp.: 130-131° C |
| 42 | 4-Br-Phenyl | H | H | c-$C_5H_9$ | Fp.: 113-115° C |
| 43 | 4-Br-Phenyl | H | H | c-$C_6H_{11}$ | Fp.: 126-128° C |
| 44 | 4-Br-Phenyl | H | H | $(CH_2)_2$-Phenyl | Fp.: 123-124° C |
| 45 | 4-Br-Phenyl | H | H | Phenyl | Fp.: 187-188° C |
| 46 | 4-Br-Phenyl | H | H | 3-F-Phenyl | Fp.: 169-170° C |

Tabelle 1 - Fortsetzung

| Bsp. Nr. | $R^1$ | $R^2$ | $R^3$ | $R^4$ | Physikalische Daten |
|---|---|---|---|---|---|
| 47 | 4-Br-Phenyl | H | H | 4-F-Phenyl | Fp.: 213-217° C |
| 48 | 4-Br-Phenyl | H | H | 4-Br-Phenyl | Fp.: 208-210° C |
| 49 | 4-Br-Phenyl | H | H | 4-$OCH_3$-Phenyl | Fp.: 206-208° C |
| 50 | 2-F-Phnyl | H | H | $C_2H_5$ | Fp.: 95-96° C |
| 51 | 4-F-Phenyl | H | H | $C_2H_5$ | Fp.: 99-100° C |
| 52 | 2,6-$F_2$-Phenyl | H | H | $C_2H_5$ | Fp.: 70-72° C |
| 53 | 3,4-$F_2$-Phenyl | H | H | c-$C_6H_{11}$ | Fp.: 63-65° C |
| 54 | 3,4-$Cl_2$-Phenyl | H | H | $C_2H_5$ | Fp.: 103-105° C |
| 55 | 2,4-$Cl_2$-Phenyl | H | H | $C_2H_5$ | Fp.: 118-119° C |
| 56 | Pyrid-4-yl | H | H | $C_2H_5$ | Fp.: 90-91° C |
| 57 | Pyrid-3-yl | H | H | $C_2H_5$ | Fp.: 66-67° C |
| 58 | Pyrid-2-yl | H | H | $C_2H_5$ | Fp.: 67-68° C |
| 59 | 2,4,6-$(CH_3)_2$-Pyrid-3-yl | H | H | $C_2H_5$ | Fp.: 75-78° C |

Tabelle 1 - Fortsetzung

| Bsp. Nr. | $R^1$ | $R^2$ | $R^3$ | $R^4$ | Physikalische Daten |
|---|---|---|---|---|---|
| 60 | 2,3-$Cl_2$-Pyrid-5-yl | H | H | $C_2H_5$ | Fp.: 154-155° C |
| 61 | Fur-2-yl | H | H | $C_2H_5$ | Fp.: 94-95° C |
| 62 | Thien-2-yl | H | H | $C_2H_5$ | Fp.: 59-60° C |
| 63 | Thien-3-yl | H | H | $C_2H_5$ | Fp.: 63-65° C |
| 64 | 5-Cl-Thien-3-yl | H | H | $C_2H_5$ | Fp.: Öl |
| 65 | 5-Br-Thien-2-yl | H | H | $C_2H_5$ | Fp.: 89-90° C |
| 66 | 4-Br-Thien-2-yl | H | H | $C_2H_5$ | Fp.: 81-83° C |
| 67 | 2,5-$(CH_3)_2$-Thien-3-yl | H | H | $C_2H_5$ | Fp.: Öl |
| 68 | 5-$CH_3$-Thien-2-yl | H | H | $C_2H_5$ | Fp.: 71-73° C |
| 69 | 4-i-$C_3H_7$-Thien-2-yl | H | H | $C_2H_5$ | Fp.: 74-75° C |
| 70 | 5-$CH_3$-Fur-2-yl | H | H | $C_2H_5$ | Fp.: 91-93° C |
| 71 | 4-Cl-Phenyl | H | H | $C_2H_5$ | Fp.: 135° C |

Beispiel 71

5-(Thien-2-yl)-4-brom-3-ethylamino-isoxazol

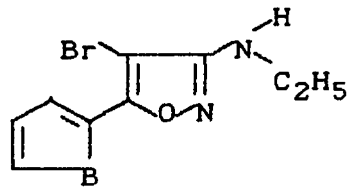

5,0 g (25,7 mmol) 5-(Thien-2-yl)-3-ethylamino-isoxazol werden in 50 ml absolutem Methylenchlorid vorgelegt und bei 5 bis 10 °C tropfenweise mit einer Lösung aus 4,5 g (28,3 mmol) Brom in 25 ml absolutem Methylenchlorid versetzt, Nach ca, 30 Minuten Rühren bei 0 bis 5 °C wird das ausgefallene kristalline Rohprodukt abgesaugt, mit Methylenchlorid gewaschen und getrocknet. Man erhält 5,2 g (74,0 % der Theorie) 5-(Thien-2-yl)-4-brom-3-ethylamino-isoxazol.

Fp.: 29-30 °C

$^1$H-NMR (DMSO-$d_6$, $\delta$): 1,18 (t, 3H, -$CH_3$; J: 7,2 Hz); 3,17 (q, 2H, -$CH_2$-; J: 7,2 Hz); 6,14 (s br., 1H, NH); 7,29; 7,77; 7,90 (3 dd, 3H, Hetarom.) ppm

Analog können die in der nachstehenden Tabelle 2 aufgeführten Verbindungen der Formel (Ib, $R^2$: -Hal) hergestellt werden.

Tabelle 2

Beispiele für Verbindungen der Formel (Ib, $R^2$: -Hal)

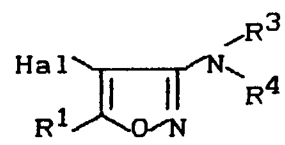

| Bsp. Nr. | $R^1$ | Hal | $R^3$ | $R^4$ | Physikalische Daten |
|---|---|---|---|---|---|
| 72 | 4-i-$C_3H_7$-Thien-2-yl | Br | H | $C_2H_5$ | Fp.: Öl |
| 73 | 4-Br-Phenyl | Br | H | $C_2H_5$ | Fp.: 124-127° C |
| 74 | 4-Br-Phenyl | Br | H | c-$C_6H_{11}$ | Fp.: 86-87° C |
| 75 | 4-F-Phenyl | Br | H | $C_2H_5$ | Fp.: 85-86° C |
| 76 | 4-Cl-Phenyl | Br | H | $C_2H_5$ | Fp.: 112-114° C |
| 77 | 4-$CH_3$-Phenyl | Br | H | $C_2H_5$ | Fp.: 82-84° C |

Ausgangsstoffe der Formel (II)

Beispiel (II-1)

(E)-1-(2,5-Dichlor-thien-3-yl)-3-ethylamino-3-methyl-thio-1-oxo-prop-2-en

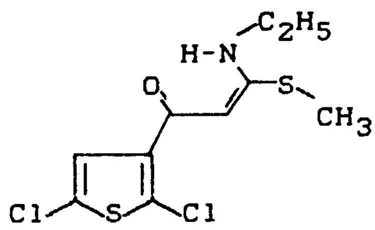

25,0 g (12,8 mmol) 3-Acetyl-2,5-dichlor-thiophen werden in 300 ml absolutem Dimethylformamid vorgelegt und bei 0 °C portionsweise mit 3,9 g (12,8 mmol) Natriumhydrid versetzt (Inertgasatmosphäre). Anschließend werden 11,2 g (12,8 mmol) Ethyl-isothiocyanat zugetropft und 2 Stunden bei Raumtemperatur gerührt. Danach gibt man unter Kühlung 18,2 g (12,8 mmol) Methyliodid hinzu, Nach 2-stündigem Rühren bei Raumtemperatur wird der gesamte Reaktionsansatz in Eiswasser eingetragen, mit Essigsäureethylester extrahiert und mit Wasser gewaschen. Die organische Phase wird über Natriumsulfat getrocknet und anschließend das Lösungsmittel abdestilliert, Das zurückbleibende Rohprodukt wird aus Diisopropylether/Ligroin umkristallisiert. Man erhält 34,7 g (91,5 % der Theorie) (E)-1-(2,5-Dichlor-thien-3-yl)-3-ethylamino-3-methylthio-1-oxo-propen.

Fp.: 53-55 °C

$^1$H-NMR ($CDCl_3$, $\delta$): 1,31 (t, 3H, $-CH_3$; J: 7,2 Hz); 2,44 (s, 3H, $-SCH_3$); 3,40 (m, 2H, $-CH_2-$; J: 7,2 Hz); 5,53 (s, 1H, =CH-); 7,09 (s, 1H, Hetarom.); 11,54 (s, 1H, N-H···O=C) ppm

Analog können die in der nachstehenden Tabelle 3 aufgeführten Verbindungen der Formel (II) hergestellt werden.

<u>Tabelle 3</u>

Ausgangsstoffe der Formel (II)

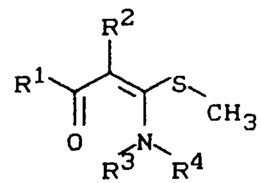

(II)

| Bsp. Nr. | $R^1$ | $R^2$ | $R^3$ | $R^4$ | Physikalische Daten |
|---|---|---|---|---|---|
| II-2 | 4-Br-Phenyl | H | H | 4-Cl-Phenyl | 2,44; 5,82; 13,45[a)] |
| II-3 | 4-Br-Phenyl | H | H | 4-$OCH_3$-Phenyl | 2,41; 5,78; 13,2[a)] |
| II-4 | 4-Br-Phenyl | H | H | c-$C_6H_{11}$ | 2,46; 5,56; 12,00[a)] |
| II-5 | 4-Br-Phenyl | H | H | 2,4-$Cl_2$-Phenyl | 2,57; 6,11; 13,5[b)] |
| II-6 | 4-Br-Phenyl | H | H | t-$C_4H_9$ | 2,48; 5,56; 12,35[a)] |
| Il-7 | 4-Br-Phenyl | H | H | $C_2H_5$ | 2,46; 5,57; 11,8[a)] |
| II-8 | 4-Br-Phenyl | H | H | i-$C_4H_9$ | 2,46; 5,58; 12,00[a)] |
| II-9 | 3,4-$(CH_3)_2$-Phenyl | H | H | $C_2H_5$ | 2,46; 5,62; 11,8[a)] |

Tabelle 3 - Fortsetzung

| Bsp. Nr. | $R^1$ | $R^2$ | $R^3$ | $R^4$ | Physikalische Daten |
|---|---|---|---|---|---|
| II-10 | 3,4-$Cl_2$-Phenyl | H | H | $C_2H_5$ | 2,47; 5,52; 11,8[a)] |
| II-11 | 2-Cl,6-F-Phenyl | H | H | $C_2H_5$ | 2,36; 5,10; 11,45[a)] |
| II-12 | 2,6-$F_2$-Phenyl | H | H | $C_2H_5$ | 2,38; 5,20; 11,55[a)] |
| II-13 | 3,4-$F_2$-Phenyl | H | H | c-$C_6H_{11}$ | 2,56; 5,72; 12,00[a)] |
| II-14 | 4-Br-Phenyl | H | H | 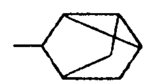 | 2,46; 5,58; 11,8[a)] |
| II-15 | 2,6-$Cl_2$-Phenyl | H | H | $C_2H_5$ | 2,36; 5,03; 11,35[a)] |
| II-16 | 2,6-$F_2$-Phenyl | H | H | c-$C_6H_{11}$ | 2,41; 5,21; 11,60[b)] |
| II-17 | 4-$CH_3$-Phenyl | H | H | $C_2H_5$ | 2,45; 5,62; 11,8[a)] |
| II-18 | 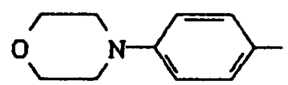 | H | H | $C_2H_5$ | 2,46; 5,61; 11,67[a)] |

<u>Tabelle 3</u> - Fortsetzung

| Bsp. Nr. | $R^1$ | $R^2$ | $R^3$ | $R^4$ | Physikalische Daten |
|---|---|---|---|---|---|
| II-19 | $3,4-(OCH_3)_2$-Phenyl | H | H | $C_2H_5$ | 2,47; 5,62; 11,68[a)] |
| II-20 | $4-N(CH_3)_2$-Phenyl | H | H | $C_2H_5$ | 2,51; 5,61; 11,62[a)] |
| II-21 | $4-SCH_3$-Phenyl | H | H | $C_2H_5$ | 2,47; 5,61; 11,75[a)] |
| II-22 | $4-OCH_3$-Phenyl | H | H | $C_2H_5$ | 2,47; 5,62; 11,70[a)] |
| II-23 | 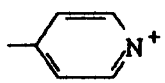 $-CH_3J^-$ | H | H | $c-C_6H_{11}$ | 2,62; 5,81; 12,27[a)] |
| II-24 | 2,3-Pyrid-5-yl | H | H | $C_2H_5$ | 2,59; 5,78; 11,77[b)] |

a) $^1$H-NMR ($CDCl_3$, δ, ppm)

b) $^1$H-NMR (DMSO-$d_6$, δ, ppm); jeweils Singuletts für $-SCH_3$; =CH-; -N-H ···O=C (H-Brücke)

Ausgangsstoffe der Formel (III)

Beispiel (III-1)

(E/Z-1-(2-Chlor-6-fluor-phenyl)-3-ethylamino-3-hydroxyamino-1-oxo-prop-2-en

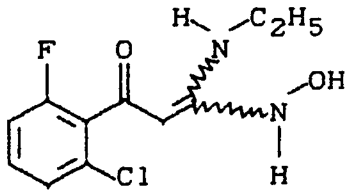

16,2 g (59,1 mmol) (E)-1-(2-Chlor-6-fluor-phenyl)-3-ethylamino-3-methylthio-1-oxo-prop-2-en werden in 3,0 ml absolutem Pyridin vorgelegt und mit 6,3 g (90,6 mmol) Hydroxylamin-hydrochlorid versetzt. Nach 2,5 Stunden wird der gesamte Reaktionsansatz in Wasser eingetragen, das ausgefallene Rohprodukt abgetrennt, mit Wasser gewaschen und aus Ethanol umkristallisiert. Man erhält 5,9 g (38,0 % der Theorie) (E/Z)-1-(2-Chlor-6-fluorphenyl)-3-ethylamino-3-hydroxyamino-1-oxo-prop-2-en.
Fp.: 203-205 ° C
$^{13}$C-NMR (DMSO-$d_6$; $\delta$): 15,1 (-$CH_3$); 36,1 (-$CH_2$-); 78,3 (-CH=); 114,6; 125,5: 129,8; 131,4 (arom. -CH); 131,5 (arom. -C-Cl); 159,0 (arom. -C-F); 161,6 (=C); 175,5 (C=O) ppm

Beispiel (III-2)

(E/Z)-1-(2,6-Dichlor-phenyl)-3-ethylamino-3-hydroxy-amino-1-oxo-prop-2-en

Herstellung analog Beispiel (III-1)
Fp.: 189-190 ° C, Ausbeute: 15,5 % der Theorie

**Patentansprüche**

1. Verwendung von 3-aminosubstituierten Isoxazolderivaten der allgemeinen Formel (I)

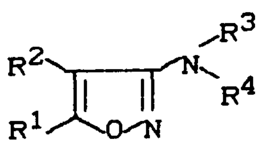

(I)

in welcher

$R^1$ für Aryl- oder Hetarylreste steht, die gegebenenfalls durch Halogen, Cyano, Nitro, Amino, Aminoacyl, Alkylamino, Alkyl, Aralkyl, Halogenalkyl, Alkoxy, Halogenalkoxy, Aryloxy, Arylthio, Halogenalkylthio, Alkylsulfinyl, Halogenalkylsulfinyl, Alkylsulfonyl, Halogenalkylsulfonyl substituiert sind,

$R^2$ für Wasserstoff, Halogen, Alkyl, Halogenalkyl, Cyano, Carbonylalkoxy, Aryl steht, die gegebenenfalls substituiert sind,

$R^3$ für substituiertes $C_1$-$C_6$-Alkyl, $C_2$-$C_4$-Alkenyl, sowie für gegebenenfalls substituiertes Cycloalkylalkyl, Cycloalkyl, Aralkyl oder Aryl steht, wobei als Substituenten genannt seien Halogen, Nitro, Alkyl, Halogenalkyl, Amino, Alkylamino, Aminoacyl, Alkoxy, Halogenalkoxy, Alkylthio, Halogenalkylthio, Alkylsulfinyl, Halogenalkylsulfinyl, Alkylsulfonyl, Halogenalkylsulfonyl, Aryl, Aryloxy, Arylthio,

$R^4$ für Wasserstoff, Alkyl steht oder $R^3$ und $R^4$ gemeinsam mit dem angrenzenden N-Atom für einen carbocyclischen 5-, 6- oder 7-gliedrigen Ring stehen, der gegebenenfalls auch durch O, S, N unterbrochen sein kann und gegebenenfalls durch $C_1$-$C_4$-Alkyl substituiert ist,

zur Bekämpfung von Endoparasiten in der Medizin und Tiermedizin.

**2.** Neue 3-aminosubstituierte Isoxazolderivate der allgemeinen Formel (I)

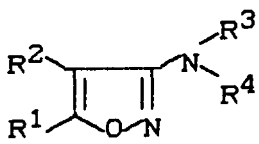

(I)

in welcher

$R^1$ für Arylreste die durch Alkoxy, Halogenalkoxy, Halogenalkylthio, Halogenalkylsulfinyl, Halogenalkylsulfonyl, sowie Hetarylreste, die gegebenenfalls durch Halogen, Alkyl, Halogenalkyl, Nitro, Alkoxy, Alkylthio substituiert sind,

$R^2$ für Wasserstoff, Halogen, Alkyl, Halogenalkyl, Cyano, Carbonylalkoxy, Aryl steht die gegebenenfalls substituiert sind,

$R^3$ für substituiertes $C_1$-$C_6$-Alkyl, $C_2$-$C_4$-Alkenyl, sowie für gegebenenfalls substituiertes Cycloalkylalkyl, Cycloalkyl, Aralkyl oder Aryl steht, wobei als Substituenten genannt seien Halogen, Nitro, Alkyl, Halogenalkyl, Amino, Alkylamino, Aminoacyl, Alkoxy, Halogenalkoxy, Alkylthio, Halogenalkylthio, Alkylsulfinyl, Halogenalkylsulfinyl, Alkylsulfonyl, Halogenalkylsulfonyl, Aryl, Aryloxy, Arylthio steht,

$R^4$ für Wasserstoff, Alkyl steht oder $R^3$ und $R^4$ gemeinsam mit dem angrenzenden N-Atom für einen carbocyclischen 5-, 6- oder 7-gliedrigen Ring stehen, der gegebenenfalls auch durch O, S, N unterbrochen sein kann und gegebenenfalls durch $C_1$-$C_4$-Alkyl substituiert ist,

ausgenommen 3-Ethylamino-5-(4-methoxyphenyl)-isoxazol.

**3.** Verfahren zur Herstellung der neuen 3-aminosubstituierten Isoxazolderivate der Formel (I)

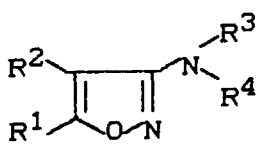

(I)

in welcher
$R^1$, $R^2$, $R^3$ und $R^4$ die in Anspruch 2 angegebene Bedeutung besitzen,
ausgenommen 3-Ethylamino-5-(4-methoxyphenyl)-isoxazol, dadurch gekennzeichnet, daß man

a) für den Fall, daß $R^2$ ausgenommen für Halogen die oben angegebene Bedeutung hat, Verbindungen der Formel (II)

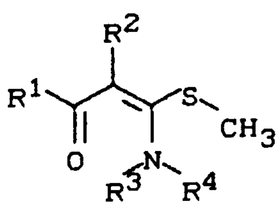

(II)

in welcher
$R^1$, $R^3$ und $R^4$ die oben angegebene Bedeutung haben,
mit Hydroxylamin umsetzt und die dabei zumeist in situ erhaltenen Verbindungen der Formel (III)

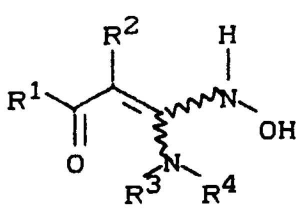

(III)

in welcher
$R^1$, $R^2$, $R^3$ und $R^4$ die oben angegebene Bedeutung haben,
in Gegenwart eines Verdünnungsmittels und gegebenenfalls in Gegenwart eines Reaktionshilfsmittels cyclisiert, oder
b) für den Fall, daß $R^2$ für Halogen steht, Verbindungen der Formel (Ia)

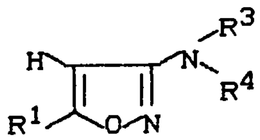

(Ia)

in welcher
$R^1$, $R^3$ und $R^4$ die unter Punkt 2 angegebene Bedeutung besitzen,
mit Halogenen der Formel (IV)

$Hal_2$ (IV)

in welcher
Hal für Halogen, vorzugsweise Fluor, Chlor, Brom oder Iod, insbesondere Brom, steht,
in Gegenwart eines Verdünnungsmittels umsetzt.

**4.** Endoparasitizide Mittel, gekennzeichnet durch einen Gehalt an mindestens einem 3-aminosubstituierten Isoxazol der Formel (I) gemäß Anspruch 1.

**5.** Verfahren zur Herstellung von endoparasitiziden Mitteln, dadurch gekennzeichnet, daß man 3-aminosubstituierte Isoxazole der Formel (I) gemäß Anspruch 1 mit Streckmitteln und/oder oberflächenaktiven Mitteln vermischt.

**6.** Verwendung von 3-aminosubstituierten Isoxazolen der Formel (I) gemäß Anspruch 1 zur Herstellung von endoparasitiziden Mitteln.

Europäisches Patentamt

# EUROPÄISCHER RECHERCHENBERICHT

Nummer der Anmeldung

EP 93 10 4387

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | SYNTHESIS.<br>Nr. 3, März 1984, STUTTGART DE<br>Seiten 247 - 249<br>A.RAHMAN ET AL 'Reaction of alpha-ketoketene S,N-acetals with hydroxylamine: a facile general route to 5-aryl-3-(N-arylamino,N-alkylamino,or N-azacycloalkyl)-isoxazoles'<br>*Seite248,249,Verbindungen 2c und 2f*<br>--- | 2,3 | C07D261/14<br>A61K31/42<br>C07D413/04<br>A61K31/44 |
| A | GB-A-2 195 628 (SHELL INTERNATIONALE RESEARCH MAATSCHAPPIJ B.V.)<br>* Seite 5 - Seite 6; Ansprüche *<br>--- | 1-3 | |
| D,A | EP-A-0 181 018 (SHELL INTERNATIONALE RESEARCH MAATSCHAPPIJ B.V.)<br>* Ansprüche *<br>* Seite 11 - Seite 14 *<br>--- | 1-3 | |
| A | US-A-3 879 532 (DUANE K. HASS ET AL)<br>* das ganze Dokument *<br>--- | 1 | **RECHERCHIERTE SACHGEBIETE (Int. Cl.5 )** |
| D,A | JOURNAL OF MEDICINAL CHEMISTRY<br>Bd. 20, Nr. 7, Juli 1977, WASHINGTON US<br>Seiten 934 - 939<br>JOHN B. CARR 'Isoxazole anthelmintics'<br>* das ganze Dokument *<br><br>----- | 1 | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 22 APRIL 1993 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)